# EUROPÄISCHE PATENTSCHRIFT

(11) **EP 2 663 260 B1**
(45) Veröffentlichungstag und Bekanntmachung des Hinweises auf die Patenterteilung: **31.10.2018**
(21) Anmeldenummer: 12700122.0
(22) Anmeldetag: 10.01.2012
(51) Int. Cl.: A61F 2/38, A61L 27/24, A61L 27/36, A61L 27/38, A61L 27/48, A61F 2/30, A61F 2/36, A61L 27/18, A61L 27/56

(54) **MEDIZINISCHES PRODUKT UND VERFAHREN ZU SEINER HERSTELLUNG, INSBESONDERE ZUR REGENERATIVEN BEHANDLUNG VON KNORPELSCHÄDEN**
MEDICAL PRODUCT AND METHOD FOR PRODUCING SAME, IN PARTICULAR FOR THE REGENERATIVE TREATMENT OF CARTILAGE DAMAGE
PRODUIT MÉDICAL ET SON PROCÉDÉ DE FABRICATION, EN PARTICULIER POUR LE TRAITEMENT RÉGÉNÉRATEUR DE DOMMAGES AU CARTILAGE

(30) Priorität: 11.01.2011 DE 102011002530
(43) Veröffentlichungstag der Anmeldung: 20.11.2013
(73) Patentinhaber: Aesculap AG, 78532 Tuttlingen (DE); TETEC Tissue Engineering Technologies AG, 72770 Reutlingen (DE)
(72) Erfinder: BOHNSACK, Claudia, 22043 Hamburg (DE); FRITZ, Jürgen, 72144 Dusslingen (DE); GAISSMAIER, Christoph, 72127 Kusterdingen (DE); MOLLENHAUER, Jürgen, 72764 Reutlingen (DE); WIESE, Dirk Hinrich, 59423 Unna (DE); STEBANI, Jürgen, 87600 Kaufbeuren (DE); MAIER, Gerhard, 80807 München (DE)
(74) Vertreter: Patentanwälte Ruff, Wilhelm, Beier, Dauster & Partner mbB
(86) Internationale Anmeldenummer: PCT/EP2012/050317
(87) Internationale Veröffentlichungsnummer: WO 2012/095428

(56) Entgegenhaltungen:
- WO-A1-96/24310
- DE-A1-102005 054 940
- US-A- 5 007 934
- US-A1- 2007 190 108

## Beschreibung

Die vorliegende Erfindung betrifft ein medizinisches Produkt, welches sich insbesondere zur Anwendung bei der Behandlung von Knorpelschäden und/oder -defekten eignet, ein Verfahren zur Herstellung des Produktes sowie verschiedene Verwendungen für das Produkt.

Knorpelgewebe stellt ein festes, druck- und biegungselastisches, gefäßloses Stützgewebe dar, welches lediglich in begrenztem Umfang zur Selbstheilung fähig ist.

Schädigungen des Knorpelgewebes können unfall- und/oder alterungsbedingt auftreten.

So unterliegt beispielsweise der Gelenkknorpel, welcher die Gelenkoberflächen als gleitfähige, wasserreiche und elastische Schicht überzieht, einem natürlichen Verschleiß. Dieser beginnt im Laufe eines menschlichen Lebens zu unterschiedlichen Zeitpunkten und ist individuell sehr unterschiedlich ausgeprägt. Das Endstadium eines derartigen Verschleißes wird üblicherweise als Arthrose bezeichnet.

Es gilt als wissenschaftlich gesichert, dass Störungen der Gelenkmechanik über einen längeren Zeitraum zu einer Knorpelschädigung führen. Solche Schäden werden beispielsweise durch eine deutliche O- oder X-Beinstellung oder auch durch einen Kreuzband- oder Meniskusschaden begünstigt. Auch Stoffwechselerkrankungen wie beispielsweise Gicht oder entzündliche rheumatische Erkrankungen können zu einer frühzeitigen Gelenkschädigung führen.

Leichte Knorpelschäden lassen sich häufig mittels konservativer Therapien wie beispielsweise mittels Verabreichung von entzündungshemmenden Medikamenten oder mittels physikalischer Therapien erfolgreich behandeln.

Liegt jedoch bereits eine fortgeschrittene Knorpelschädigung, insbesondere in Form einer Arthrose, vor, kommen in der Regel ausschließlich operative Maßnahmen zur Anwendung. Abhängig vom Schweregrad der Knorpelschädigung stehen dabei unterschiedliche Behandlungsmaßnahmen zur Verfügung.

Eine in neuerer Zeit in zunehmendem Maße angewandte Behandlungsmethode stellt die sogenannte autologe Chondrozytentransplantation (ACT) dar. Bei dieser Methode werden in einer ersten Operation arthroskopisch autologe Knorpelzellen (Chondrozyten) entnommen. Die Knorpelzellen werden im Labor angezüchtet und in einer zweiten Operation einige Wochen später unter einen den Knorpeldefekt verschließenden Knochenhautlappen (Periostlappen) oder unter eine spezielle Membran eingespritzt.

Bei einer Variante der autologen Chondrozytentransplantation, der sogenannten matrixgestützten autologen Chondrozytentransplantation (MACT), werden die entnommenen Knorpelzellen auf einer geeigneten dreidimensionalen resorbierbaren Matrix gezüchtet. Anschließend wird die Matrix in den Knorpeldefekt eingenäht und vom Körper innerhalb weniger Wochen abgebaut. Eine Deckelung mit Knochenhaut ist bei diesem Verfahren nicht notwendig, was die Operation wesentlich erleichtert.

Da bei der ACT bzw. MACT die Chondrozyten wegen des aufgenähten Knochenhautlappens bzw. der Matrix daran gehindert werden, den Knorpeldefekt vorzeitig zu verlassen, lassen sich mittels dieser Verfahren grundsätzlich auch größere Knorpeldefekte behandeln. Allerdings erweisen sich sowohl die ACT als auch die MACT bei der Behandlung von korrespondierenden Gelenkflächen wie beispielsweise beim Kniegelenk nur begrenzt tauglich. Hinzu kommt, dass bei der ACT der Periostlappen im Prinzip wasserdicht am Rand eines noch vorhandenen gesunden Knorpels angenäht werden muss.

Weiterhin erweist sich die Fixierung von dreidimensionalen Matrices in Knorpeldefektzonen als grundsätzlich schwierig und aufwendig. So müssen die Matrices entlang ihrer Ränder entweder mit noch vorhandenem natürlichem Knorpel vernäht oder aber mittels geeigneter Ankerelemente im Knochen fixiert werden. Abhängig vom Material der Matrices kann es darüber hinaus bei Einwirkung größerer Belastungskräfte im Körper eines Patienten zu einem Ausreißen von Nähten und damit zu einer unerwünschten Dislokation der Matrices kommen.

Eine weitere Schwierigkeit besteht darin, eine möglichst schnelle und insbesondere homogene zelluläre Besiedelung der Matrices zu realisieren, um den Heilungsprozess bzw. die Neubildung von Knorpelgewebe voranzutreiben.

Aus der US 5,007,934 ist ein Meniskusimplantat mit einer porösen Matrix aus bioverträglichen und bioresorbierbaren Fasern bekannt.

Gegenstand der US 2007/0190108 A1 ist ein Implantat mit einer elastomeren Matrix sowie einer Verstärkungskomponente.

Die WO 96/24310 A1 offenbart ein schichtförmig aufgebautes Implantat mit einer Kollagenmembran sowie einer porösen Kollagenmatrix.

Aufgabe der vorliegenden Erfindung ist es daher, ein medizinisches Produkt bereitzustellen, welches sich insbesondere zur Regeneration bzw. Rekonstruktion von Knorpelgewebe jeglicher Art eignet und dabei aus dem Stand der Technik bekannte Nachteile möglichst umgeht. Das medizinische Produkt soll insbesondere rasch und vorzugsweise homogen mit Zellen besiedelbar sein und zudem eine möglichst positionsfeste Verankerung bzw. Fixierung in einer menschlichen oder tierischen Körperdefektzone ermöglichen. Darüber hinaus soll das Produkt Belastungskräften, wie sie typischerweise im menschlichen oder tierischen Körper auftreten, standhalten können, ohne dass es hierdurch zu einer Beschädigung des Produktes kommt.

Diese Aufgabe wird erfindungsgemäß gelöst durch ein medizinisches Produkt, insbesondere zur Anwendung bei der Behandlung, vorzugsweise regenerativen Behandlung, von Knorpelschäden und/oder -defekten, umfassend eine schaumartige Komponente, eine membranartige Komponente und eine faserförmige Komponente.

Insbesondere kann das Produkt aus einer schaumartigen Komponente, membranartigen Komponente und faserförmigen Komponente bestehen.

Unter einer schaumartigen Komponente soll im Sinne der vorliegenden Erfindung eine Komponente mit einer schaumartigen bzw. schaumähnlichen Struktur oder einer Schaumstruktur verstanden werden.

Unter einer membranartigen Komponente soll im Sinne der vorliegenden Erfindung eine Komponente mit einer membranartigen bzw. membranähnlichen Struktur oder einer Membranstruktur verstanden werden.

Unter einer Körperdefektzone soll im Sinne der vorliegenden Erfindung bevorzugt eine Gewebsdefektzone, weiter bevorzugt eine Bindegewebsdefektzone, besonders bevorzugt eine Stützgewebsdefektzone, höchst bevorzugt eine Knorpeldefektzone, insbesondere eine Meniskusdefektzone, verstanden werden.

Unter einem Zielgewebe soll bevorzugt ein mit Hilfe des erfindungsgemäßen Produktes zu regenerierendes bzw. zu rekonstruierende Gewebe, weiter bevorzugt Bindegewebe, besonders bevorzugt Stützgewebe, höchst bevorzugt Knorpelgewebe, insbesondere Meniskusgewebe, verstanden werden.

Entsprechend sollen unter Zellen, sofern nicht anders beschrieben, vor allem Bindegewebszellen, insbesondere Stützgewebszellen, bevorzugt Knorpelzellen (Chondrozyten), Stromazellen und/oder Stammzellen, insbesondere mesenchymale Stromazellen und/oder mesenchymale Stammzellen, verstanden werden.

Mit besonderem Vorteil ist das medizinische Produkt, insbesondere die schaumartige Komponente, die membranartige Komponente und/oder die faserförmige Komponente, bioverträglich bzw. biokompatibel, insbesondere gegenüber Zellen.

Des Weiteren ist es von Vorteil, wenn das medizinische Produkt, insbesondere die schaumartige Komponente, die membranartige Komponente und/oder die faserförmige Komponente, resorbierbar, insbesondere bioresorbierbar, d.h. in vivo resorbierbar, ist.

Die schaumartige Komponente ist in einer bevorzugten Ausführungsform aus einem Polymer, insbesondere Homopolymer, Heteropolymer, Copolymer und/oder einer Mischung, insbesondere einem Blend, aus verschiedenen Polymeren, gebildet. Bei dem Polymer handelt es sich dabei in der Regel um ein synthetisches Polymer, insbesondere einen Kunststoff. Vorzugsweise ist das Polymer resorbierbar, insbesondere bioresorbierbar, ausgebildet.

In einer bevorzugten Ausführungsform weist die schaumartige Komponente ein geschäumtes Polymer oder gegebenenfalls eine geschäumte Polymermischung auf. Unter einem geschäumten Polymer bzw. einer geschäumten Polymermischung soll im Sinne der vorliegenden Erfindung ein Polymer bzw. eine Polymermischung verstanden werden, welches bzw. welche mittels dem Fachmann an sich bekannten Schäumungsverfahren wie beispielsweise des Reaktionsschaumguss-Verfahrens oder nach Art eines Reaktionsschaumguss-Verfahrens als Schaum oder schaumartige Matrix bzw. Struktur hergestellt werden kann.

In einer besonders bevorzugten Ausführungsform handelt es sich bei der schaumartigen Komponente um einen Polymerschaum, insbesondere um einen synthetischen Polymerschaum, bevorzugt um einen Kunststoffschaum.

In einer weitergehenden Ausführungsform handelt es sich bei der schaumartigen Komponente um ein geschäumtes Polymer, insbesondere um ein geschäumtes synthetisches Polymer, bevorzugt um einen geschäumten Kunststoff.

Das Polymer, insbesondere wie es in den vorhergehenden Ausführungsformen beschrieben worden ist, ist in einer weiteren Ausführungsform ein Polyaddukt, insbesondere hergestellt durch eine nukleophile Addition zwischen wenigstens zwei verschiedenartigen polyfunktionellen, insbesondere bifunktionellen, Monomertypen.

Gemäß einer besonders bevorzugten Ausführungsform handelt es sich bei dem Polymer um Polyurethan. Polyurethan ist aufgrund seiner bioverträglichen Eigenschaften ein an sich geschätztes Material auf dem Gebiet der Medizintechnik.

Das Polymer kann insbesondere ein lineares oder verzweigtes Polyurethan sein. Zusätzlich oder alternativ kann das Polymer ein aliphatisches oder aromatisches Polyurethan sein. Ein lineares und insbesondere aliphatisches Polyurethan kann bevorzugt sein.

Das Polymer ist in einer weiteren Ausführungsform ein Polyurethan, welches ausgehend von wenigstens einer Polyolkomponente und wenigstens einer Polyisocyanatkomponente, bevorzugt mittels eines Schäumungsverfahrens, insbesondere mittels eines Reaktionsschaumguss-Verfahrens bzw. nach Art eines Reaktionsschaumguss-Verfahrens, hergestellt bzw. herstellbar ist.

Unter einer Polyolkomponente soll im Sinne der vorliegenden Erfindung eine Alkoholverbindung mit zwei Hydroxygruppen (Diolkomponente) oder mehr Hydroxygruppen verstanden werden. In entsprechender Weise soll unter einer Polyisocyanatkomponente im Sinne der vorliegenden Erfindung eine Isocyanatverbindung mit zwei Isocyanatgruppen (Diisocyanatkomponente) oder mehr Isocyanatgruppen verstanden werden.

Die wenigstens eine Polyolkomponente und/oder die wenigstens eine Polyisocyanatkomponente können als Monomere, Oligomere und/oder Polymere vorliegen. Weiterhin ist es bevorzugt, wenn die Polyolkomponente und/oder die Polyisocyanatkomponente flüssig bzw. verflüssigbar sind, bevorzugt in einem Temperaturbereich zwischen 20 und 45 °C, insbesondere 25 und 40 °C.

Die wenigstens eine Polyolkomponente und/oder die wenigstens eine Polyisocyanatkomponente können weiterhin linear und/oder verzweigt sein. Außerdem können die wenigstens eine Polyolkomponente und/oder die wenigstens eine Polyisocyanatkomponente aliphatisch und/oder aromatisch sein.

In einer besonders bevorzugten Ausführungsform ist das Polymer ein Polyurethan, welches ausgehend von einer Polyolkomponente, welche ausgewählt ist aus der Gruppe bestehend aus Ethylenglykol, Diethylenglykol, Propylenglykol, Trimethylolpropan, Glycerin, Neopentylglykol, 1,3-Propandiol, 1,4-Butandiol, 1,6-Hexandiol, Polycaprolactondiol, Polycaprolactontriol, Polyethylenglykol und Mischungen davon, und einer Polyisocyanatkomponente, welche ausgewählt ist aus der Gruppe bestehend aus Diphenylmethandiisocyanat, Toluol-2,4-diisocyanat, Naphthylendiisocyanat, Hexamethylendiisocyanat, Isophorondiisocyanat, 4,4'-Diisocyanatodicyclohexylmethan, Butandiisocyanat, Lysindiisocyanat und Mischungen davon, durch nukleophile Polyaddition hergestellt bzw. herstellbar ist.

Die schaumartige Komponente weist vorzugsweise eine poröse, insbesondere offenporöse, Struktur auf. Dies begünstigt mit besonderem Vorteil ein Einwachsen bzw. Einsprossen von Zellen, Gewebe und/oder natürlichen Versorgungsgefäßen wie beispielsweise Blutgefäßen in das Innere der schaumartigen Komponente.

Bevorzugt besitzt die schaumartige Komponente eine Porengröße zwischen 10 und 450 µm, insbesondere 30 und 350 µm, vorzugsweise 50 und 250 µm. Diese Porengrößen haben sich als besonders vorteilhaft im Hinblick auf das Einwachsen bzw. Einsprossen von Zellen, Gewebe und Versorgungsgefäßen erwiesen.

Die schaumartige Komponente weist weiterhin vorzugsweise eine Porosität zwischen 65 und 85 %, insbesondere 65 und 80 %, auf.

In einer weiteren Ausführungsform besitzt die schaumartige Komponente Bereiche mit unterschiedlicher Porenstruktur, insbesondere unterschiedlichen Porengrößen und/oder -geometrien.

Insbesondere kann die schaumartige Komponente einen Porengradienten besitzen.

In einer weiteren Ausführungsform weist die schaumartige Komponente eine oberschenkelseitige Oberfläche, eine schienbeinseitige Oberfläche und eine gelenkkapselseitige Oberfläche auf. Unter einer oberschenkelseitigen Oberfläche soll im Sinne der vorliegenden Erfindung eine Oberfläche der schaumartigen Komponente verstanden werden, welche nach der Implantation des Produktes in ein Kniegelenk dem Oberschenkel (Femur) bzw. den Oberschenkel-Kondylen (femoralen Kondylen) zugewandt ist. Eine schienbeinseitige Oberfläche soll im Sinne der vorliegenden Erfindung eine Oberfläche der schaumartigen Komponente bedeuten, welche nach der Implantation des Produktes in ein Kniegelenk dem Schienbein (Tibia) bzw. den Schienbein-Kondylen (tibialen Kondylen) zugewandt ist. Unter einer gelenkkapselseitigen Oberfläche soll im Sinne der vorliegenden Erfindung eine Oberfläche der schaumartigen Komponente verstanden werden, welche nach der Implantation des Produktes der Gelenkkapsel (Randleiste) des Kniegelenks zugewandt ist.

Erfindungsgemäß kann es beispielsweise vorgesehen sein, dass die schaumartige Komponente in Richtung der oberschenkelseitigen Oberfläche und/oder in Richtung der schienbeinseitigen Oberfläche abnehmende Porengrößen aufweist. Insbesondere können die oberschenkelseitige Oberfläche und/oder die schienbeinseitige Oberfläche derart kleine Porengrößen aufweisen, dass eine zelluläre Einsprossung nicht mehr möglich ist. In der Regel handelt es sich hierbei um Porengrößen < 1 µm. Derart kleine Porengrößen verleihen der oberschenkelseitigen Oberfläche und/oder der schienbeinseitigen Oberfläche gleitfähigere Eigenschaften, wodurch eine reibungslosere Artikulation mit dem Oberschenkelknochen (Femur) und/oder dem Schienbeinknochen (Tibia) möglich ist.

In einer weitergehenden Ausführungsform ist lediglich die gelenkkapselseitige Oberfläche der schaumartigen Komponente offenporös ausgebildet. Insbesondere weist nur die gelenkkapselseitige Oberfläche der schaumartigen Komponente Porengrößen auf, die eine zelluläre Einsprossung in die schaumartige Komponente erlauben. Die übrigen Oberflächen der Schaumkomponente, insbesondere eine oberschenkelseitige Oberfläche und/oder eine schienbeinseitige Oberfläche, sind dagegen vorzugsweise nicht porös ausgebildet oder aber weisen Porengrößen auf, die eine zelluläre Einsprossung in die schaumartige Komponente nicht erlauben. Wie bereits erwähnt, handelt es sich hierbei in der Regel um Poren mit einer Porengröße < 1 µm.

In einer weiteren Ausführungsform weist die schaumartige Komponente einen Anteil zwischen 70 und 98 Gew.-%, insbesondere 80 und 97 Gew.-%, vorzugsweise 85 und 95 Gew.-%, auf, bezogen auf das Gesamtgewicht des Produktes.

Bevorzugt weist die schaumartige Komponente eine Dichte zwischen 0,17 und 0,45 g/cm³, bevorzugt zwischen 0,24 und 0,42 g/cm³, auf.

Die schaumartige Komponente weist in einer besonders vorteilhaften Ausführungsform Kanäle bzw. kanalartige Strukturen auf. Die Kanäle bzw. kanalartigen Strukturen können einen Innendurchmesser (lichte Weite) zwischen 100 und 600 µm, insbesondere 200 und 500 µm, bevorzugt von ca. 500 µm, aufweisen. Die Kanäle bzw. kanalartigen Strukturen stellen mit besonderem Vorteil Leit- oder Führungsschienen für einsprossende Zellen, einsprossendes Gewebe und/oder einsprossende Versorgungsgefäße, insbesondere Blutgefäße, dar.

Des Weiteren ist es bevorzugt, wenn die Kanäle bzw. kanalartigen Strukturen eine poröse Innenwandung aufweisen. Dadurch ist eine vorzugsweise vollständige Erschließung der schaumartigen Komponente durch einwachsende Zellen, einwachsendes Gewebe und/oder einwachsende Versorgungsgefäße erzielbar.

In einer weitergehenden Ausführungsform sind die Kanäle bzw. kanalartigen Strukturen in einem peripheren Bereich der schaumartigen Komponente und gegebenenfalls in einem an den peripheren Bereich angrenzenden mittleren Bereich der schaumartigen Komponente ausgebildet. Bevorzugt ist ein an den mittleren Bereich angrenzender zentraler Bereich der schaumartigen Komponente frei von Kanälen bzw. kanalartigen Strukturen.

Die membranartige Komponente erlaubt vorzugsweise eine sichere und insbesondere verschiebefeste bzw. dislokationsfeste Verankerung des medizinischen Produktes in einer Körperdefektzone. Die membranartige Komponente ist insbesondere in der Lage, auf die schaumartige Komponente einwirkende Kräfte, beispielsweise Scherkräfte, wie sie typischerweise im Körper eines Patienten auftreten können, wenigstens teilweise abzufedern, um dadurch einer mechanischen Beschädigung der schaumartigen Komponente vorzubeugen.

Erfindungsgemäß ist es daher besonders bevorzugt, wenn die membranartige Komponente eine reiß- bzw. zugfeste Struktur besitzt.

Bevorzugt besitzt die membranartige Komponente eine Reißfestigkeit zwischen 15 und 150 N/cm, insbesondere 20 und 100 N/cm, vorzugsweise 30 und 50 N/cm.

Erfindungsgemäß kann es weiterhin von Vorteil sein, wenn die membranartige Komponente eine Barriere für Zellen, insbesondere für unspezifische Bindegewebszellen wie beispielsweise Fibroblasten, darstellt bzw. - in anderen Worten - eine für Zellen undurchlässige Struktur besitzt. Dadurch kann die membranartige Komponente mit besonderem Vorteil ebenfalls als eine Art Leit- bzw. Führungsschiene für Zellen wirken, indem sie beispielsweise das Einsprossen von Zellen in die schaumartige Komponente und/oder die Migration von Zellen innerhalb der schaumartigen Komponente kanalisiert und insbesondere verstärkt. Dadurch ist eine schnelle, quantitative und insbesondere homogene zelluläre Besiedelung der schaumartigen Komponente erzielbar. Dieser Effekt ist sowohl im Hinblick auf eine in vitro Kultivierung des Produktes mit Zellen, insbesondere autologen Körperzellen, als auch im Hinblick auf eine zelluläre Einsprossung in die schaumartige Komponente nach Implantation des Produktes in eine Körperdefektzone von Vorteil. Im Ergebnis stehen daher mehr Zellen für die Rekonstruktion eines Zielgewebes zur Verfügung, wodurch sich die Heilungschancen für den Patienten deutlich verbessern.

Des Weiteren kann es von Vorteil sein, wenn die membranartige Komponente auch eine Leitschienenfunktion in Bezug auf Gewebe wie beispielsweise peripheres Bindegewebe ausübt. Dadurch ist eine natürliche bzw. native Verankerung des erfindungsgemäßen Produktes im Körper eines Patienten, insbesondere mit umliegenden Körpergewebebereichen einer Körperdefektzone, realisierbar.

Weiterhin kann es von Vorteil sein, wenn die membranartige Komponente auch eine Leitschienenfunktion gegenüber natürlichen Versorgungsgefäßen wie beispielsweise Blutgefäßen übernimmt. Durch das Einsprossen von Versorgungsgefäßen in die schaumartige Komponente kann eine ausreichende Versorgung von in der schaumartigen Komponente enthaltenen Zellen mit Nährstoffen sichergestellt werden. Dadurch lässt sich die Neubildung bzw. Rekonstruktion von Zielgewebe ebenfalls beschleunigen, was sich ebenfalls wiederum günstig auf den Behandlungserfolg auswirkt.

Um eine Leitschienenfunktion ausüben zu können, kann die membranartige Komponente eine nicht poröse bzw. eine im Wesentlichen nicht poröse Struktur aufweisen. Alternativ kann die membranartige Komponente Poren besitzen, deren Größe (lichte Weite bzw. Innendurchmesser) kleiner als der Durchmesser von Zellen ist. Erfindungsgemäß ist es daher denkbar, dass die membranartige Komponente Poren mit einer Größe < 1 µm, insbesondere < 0,5 µm, aufweist.

Die membranartige Komponente ist in einer weiteren Ausführungsform flächig, insbesondere zweidimensional, blattartig, hautartig, folienartig oder schichtförmig ausgebildet.

Die membranartige Komponente kann des Weiteren eine Schichtdicke zwischen 50 und 2.000 µm, insbesondere 100 und 1.500 µm, vorzugsweise 150 und 1.000 µm, aufweisen.

In einer weiteren Ausführungsform weist die membranartige Komponente einen Anteil zwischen 1,5 und 20 Gew.-%, insbesondere 2 und 15 Gew.-%, vorzugsweise 2 und 10 Gew.-%, auf, bezogen auf das Gesamtgewicht des Produktes.

Die membranartige Komponente ist teilweise, insbesondere nur teilweise, in der schaumartigen Komponente enthalten bzw. liegt teilweise, insbesondere nur teilweise, eingebettet in der schaumartigen Komponente vor.

Insbesondere kann die membranartige Komponente teilweise, insbesondere nur teilweise, in die Struktur der schaumartigen Komponente integriert sein.

In einer besonders bevorzugten Ausführungsform ragt die membranartige Komponente in die schaumartige Komponente bzw. deren Struktur hinein. Mit anderen Worten ist es besonders bevorzugt, wenn die membranartige Komponente aus der schaumartigen Komponente bzw. deren Struktur herausragt bzw. wenn die membranartige Komponente teilweise nicht von der schaumartigen Komponente umgeben oder umhüllt ist.

Weiterhin ist es bevorzugt, wenn sich die membranartige Komponente, vorzugsweise durchgehend, in Längsrichtung der schaumartigen Komponente erstreckt.

Bevorzugt ragt die membranartige Komponente, vorzugsweise in Längsrichtung der schaumartigen Komponente, entlang einer gelenkkapselseitigen Oberfläche der schaumartigen Komponente aus letzterer heraus.

Erfindungsgemäß kann es beispielsweise vorgesehen sein, dass ca. zwei Drittel der membranartigen Komponente aus der schaumartigen Komponente herausragen und ca. ein Drittel der membranartigen Komponente in die schaumartige Komponente hineinragen.

Die vorhergehenden, die räumliche Beziehung zwischen der schaumartigen Komponente und der membranartigen Komponente näher beschreibenden Ausführungsformen haben insbesondere die folgenden Vorteile:
- Bei der Verankerung des medizinischen Produktes in einer Köperdefektzone können Nahtmaterialien oder Fäden nicht nur durch die schaumartige Komponente, sondern auch durch die membranartige Komponente hindurchgeführt werden, wodurch im Ergebnis eine festere Verankerung des Produktes erzielbar und insbesondere das Risiko von unerwünschten Dislokationen während der Heilungsphase und einer Gewebeneubildung drastisch reduzierbar ist. Insbesondere kann auf diese Weise auch bei Einwirkung größerer Kräfte auf das Produkt ein Ausreißen von Fäden, welche zur Verankerung des Produktes verwendet worden sind, aus der schaumartigen Komponente und damit eine mechanische Beschädigung derselben vermieden werden.
- Ist die membranartige Komponente über die gesamte Länge der schaumartigen Komponente ausgebildet, können auf die schaumartige Komponente einwirkende Kräfte über deren gesamte Länge besser abgefedert bzw. aufgenommen werden, ohne dass die schaumartige Komponente hierdurch beschädigt wird.
- Des Weiteren kann der aus der schaumartigen Komponente herausragende Teil der membranartigen Komponente mit besonderem Vorteil als eine Art Leitschiene für Zellen aus einem Kultivierungsmedium oder aus Körpergewebebereichen in Richtung der schaumartigen Komponente wirken. Auf diese Weise ist eine schnelle und insbesondere quantitative zelluläre Kolonisierung der schaumartigen Komponente möglich.
- Der in die schaumartige Komponente hineinragende Teil der membranartigen Komponente kann mit besonderem Vorteil als eine Art Leitschiene für bereits in der schaumartigen Komponente enthaltene Zellen wirken. Die Zellen können sich entlang der membranartigen Komponente vorwärtsbewegen und auf diese Weise in alle Bereiche der schaumartigen Komponente, insbesondere in deren Mitte und/oder Endabschnitte, vordringen. Auf diese Weise ist eine vorzugsweise homogene zelluläre Besiedelung der schaumartigen Komponente möglich.
- Die beiden zuletzt beschriebenen Vorteile treffen in entsprechender Weise auch für die Leitschienenfunktion der membranartigen Komponente zu, welche diese vorzugsweise gegenüber Geweben wie beispielsweise peripheres Bindegewebe und/oder natürlichen Versorgungsgefäßen wie beispielsweise Blutgefäßen ausübt. Dies ist vor allem bei der Behandlung von Meniskusschäden von Vorteil, da der faserige Anteil eines Meniskus nicht nur durch Diffusion, sondern - wie andere Sehnen und Bänder auch - durch Blutversorgung mit Nährstoffen versorgt wird und über faseriges Bindegewebe fest im menschlichen Körper verankert ist.

In einer weiteren Ausführungsform besitzt die membranartige Komponente Durchbrechungen, insbesondere in Form von Öffnungen, Löcher, Perforationen oder dergleichen. Entlang der Durchbrechungen ist die membranartige Komponente vorzugsweise von der schaumartigen Komponente durchsetzt bzw. durchdrungen. Dadurch ist eine feste Integration der membranartigen Komponente in der schaumartigen Komponente erzielbar.

Die membranartige Komponente ist tierischen Ursprungs. Die membranartige Komponente kann beispielsweise porcinen, bovinen und/oder equinen Ursprungs sein. Bevorzugt ist die membranartige Komponente bovinen Ursprungs.
Die membranartige Komponente weist in einer weiteren Ausführungsform eine faserförmige Struktur oder eine Faserstruktur auf.

Vorzugsweise weist die membranartige Komponente ein Protein, insbesondere ein fibrilläres oder faserförmiges Protein, bevorzugt ein extrazelluläres Protein, auf.

Das Protein ist bevorzugt als Hauptbestandteil in der membranartigen Komponente enthalten. So kann die membranartige Komponente einen Proteinanteil von mehr als 50 Gew.-%, insbesondere von mehr als 75 Gew.-%, bevorzugt von mehr als 90 Gew.-%, aufweisen, bezogen auf das Gesamtgewicht der membranartigen Komponente.

Vorzugsweise ist die membranartige Komponente aus dem Protein gebildet.

Das Protein kann insbesondere ausgewählt sein aus der Gruppe bestehend aus Kollagen, Elastin, Retikulin, Fibronektin, Gelatine, Salze davon, Derivate davon und Mischungen davon.

In einer bevorzugten Ausführungsform ist das Protein Kollagen, insbesondere faserförmiges bzw. fibrilläres Kollagen, vorzugsweise ausgewählt aus der Gruppe bestehend aus Kollagen vom Typ I, Kollagen vom Typ II, Kollagen vom Typ III, Kollagen vom Typ V, Kollagen vom Typ XI, Salze davon, Derivate davon und Mischungen davon. Kollagen vom Typ I ist erfindungsgemäß besonders bevorzugt.

Bei der membranartigen Komponente handelt es sich um eine Membran. Besonders bevorzugt handelt es sich bei der membranartigen Komponente um eine Proteinmembran, insbesondere Kollagenmembran. Eine besonders bevorzugte membranartige Komponente ist die von der Anmelderin kommerziell unter der Bezeichnung Lyoplant® vertriebene Kollagenmembran. Bezüglich weiterer Merkmale und Vorteile zu dem Protein, insbesondere Kollagen, wird auf die bisherigen Ausführungen vollständig Bezug genommen.

In einer weiteren Ausführungsform handelt es sich bei der membranartigen Komponente um ein biologisches Gewebe, insbesondere um eine Gewebsschicht wie beispielsweise die Submucosa, oder eine Haut wie beispielsweise eine seröse Haut. Unter einem biologischen Gewebe soll im Sinne der vorliegenden Erfindung ein Gewebe verstanden werden, welches in tierischen Organismen vorkommt.

In einer weiteren vorteilhaften Ausführungsform handelt es sich bei der membranartigen Komponente um eine extrazelluläre Matrix (ECM), insbesondere ausgewählt aus der Gruppe bestehend aus Perikard, Peritoneum, Dünndarm-Submucosa, Magen-Submucosa, Harnblasen-Submucosa, Uterus-Submucosa, Serosa und Kombinationen davon.

In einer bevorzugten Ausführungsform ist die membranartige Komponente ausgewählt aus der Gruppe bestehend aus Herzbeutel bzw. Perikard, Pericardium fibrosum, Pericardium serosum, Epikard, Plattenepithel, Tunica serosa, Muskel wie beispielsweise Myokard und Kombinationen davon.

In einer besonders bevorzugten Ausführungsform handelt es sich bei der membranartigen Komponente um Perikard, insbesondere tierisches Perikard. Das Perikard kann beispielsweise porcinen, bovinen und/oder equinen Urspungs sein. Vorzugsweise handelt es sich bei der membranartigen Komponente um bovines Perikard bzw. Rinderperikard.

Die faserförmige Komponente kann ebenfalls als Leit- bzw. Führungsschiene für in die schaumartige Komponente einwachsende bzw. einsprossende Zellen wirken. Dadurch ist ebenfalls eine schnelle und insbesondere homogene Kolonisierung der schaumartigen Komponente mit Zellen erzielbar. Darüber hinaus kann die Porosität einer porösen schaumartigen Komponente durch die faserförmige Komponente zusätzlich erhöht werden, was sich wiederum vorteilhaft auf die zelluläre Einsprossung in die schaumartige Komponente auswirkt. Des Weiteren kann auch die faserförmige Komponente das Einwachsen von Gewebe, insbesondere peripherem Bindegewebe, und/oder Versorgungsgefäßen wie beispielsweise Blutgefäßen in die schaumartige Komponente begünstigen.

Die faserförmige Komponente ist vorzugsweise in der schaumartigen Komponente, insbesondere ausschließlich in der schaumartigen Komponente, enthalten.

Bevorzugt weist die faserförmige Komponente das gleiche Material auf wie die membranartige Komponente bzw. ist aus dem gleichen Material gebildet wie die membranartige Komponente.

In einer vorteilhaften Ausführungsform handelt es sich bei der faserförmigen Komponente um hydrophobe Fasern. Hydrophobe Fasern haben den Vorteil, dass sich Zellen besser daran anhaften können, wodurch sich im Ergebnis die Leitschienenfunktion für Zellen verbessern lässt.

Bei der faserförmigen Komponente handelt es sich in einer weiteren Ausführungsform um biopolymerhaltige, insbesondere polysaccharidhaltige wie beispielsweise mucopolysaccharidhaltige und/oder proteinhaltige, vorzugsweise kollagenhaltige, Fasern.

Bevorzugt handelt es sich bei der faserförmigen Komponente um Fasern mit einem Proteinanteil, vorzugsweise Kollagenanteil, insbesondere einem Anteil an Kollagen vom Typ I, von mehr als 50 Gew.-%, insbesondere von mehr als 60 Gew.-%, bevorzugt von mehr als 70 Gew.-%, besonders bevorzugt von ca. 75 Gew.-%, bezogen auf das Einzelgewicht der Fasern.

Bezüglich weiterer Merkmale zu dem Protein, vorzugsweise Kollagen, wird vollständig auf die bisherigen, im Zusammenhang mit der membranartigen Komponente gemachten Ausführungen Bezug genommen.

In einer weiteren Ausführungsform handelt es sich bei der faserförmigen Komponente um Fasern biologischen, insbesondere tierischen, Ursprungs. Beispielsweise kann es sich bei der faserförmigen Komponente um Fasern porcinen, bovinen und/oder equinen Ursprungs handeln. Bovine Fasern sind besonders bevorzugt.

In einer bevorzugten Ausführungsform handelt es sich bei der faserförmigen Komponente um Lyoplant®-Fasern.

In einer weiteren bevorzugten Ausführungsform handelt es sich bei der faserförmigen Komponente um Perikardfasern, Epikardfasern, Myokardfasern oder Kombinationen davon.

Die faserförmige Komponente weist bevorzugt einen Anteil zwischen 1 und 10 Gew.-%, insbesondere 1,5 und 7,5 Gew.-%, vorzugsweise 2 und 6 Gew.-%, auf, bezogen auf das Gesamtgewicht des Produktes.

Das Produkt, insbesondere die schaumartige Komponente und/oder die membranartige Komponente, weist in einer weiteren vorteilhaften Ausführungsform Zellen, insbesondere autologe Zellen, bevorzugt autologe Körperzellen, auf. Die Zellen können ausgewählt sein aus der Gruppe bestehend aus Chondrozyten, Osteoblasten, Zellen aus dem Periostgewebe, Zellen aus dem Perichondriumgewebe, Synovialzellen, Fibroblasten, Stromazellen, insbesondere mesenchymale Stromazellen, Vorläuferzellen davon, Stammzellen davon, Stammzellen, insbesondere mesenchymale Stammzellen, und Mischungen davon.

Das Produkt, insbesondere die schaumartige Komponente und/oder die membranartige Komponente, weist in einer weitergehenden Ausführungsform Bereiche mit unterschiedlicher Zelldichte auf.

Bevorzugt weist das Produkt, insbesondere die schaumartige Komponente und/oder die membranartige Komponente, einen peripheren Bereich, einen an den peripheren Bereich angrenzenden mittleren Bereich und einen an den mittleren Bereich angrenzenden zentralen Bereich auf.

Erfindungsgemäß kann es beispielsweise vorgesehen sein, dass der zentrale Bereich eine höhere Zelldichte aufweist als der periphere Bereich und/oder der mittlere Bereich, insbesondere als der periphere Bereich und der mittlere Bereich. Eine erhöhte Zelldichte im zentralen Bereich ist insbesondere bei einem als Meniskusersatz- oder Meniskusteilersatzimplantat für das Kniegelenk ausgebildeten medizinischen Produkt von Vorteil, da der zentrale Bereich in diesem Fall besonders hohen Belastungen ausgesetzt und eine beschleunigte Neubildung von Knorpelgewebe gerade in diesem Bereich unter medizinischorthopädischen Gesichtspunkten besonders wünschenswert ist.

In einer weiteren Ausführungsform weist das Produkt, insbesondere die schaumartige Komponente und/oder die membranartige Komponente, Wirkstoffe, bevorzugt biologische und/oder pharmazeutisch bzw. medizinisch wirksame Wirkstoffe, auf.

Die Wirkstoffe können ausgewählt sein aus der Gruppe bestehend aus antimikrobielle, insbesondere antibiotische, Wirkstoffe, desinfizierende Wirkstoffe, schmerzlindernde Wirkstoffe, geruchsbekämpfende Wirkstoffe, das Zellwachstum fördernde Wirkstoffe, Gewebewachstum, insbesondere Knorpelwachstum, fördernde Wirkstoffe, die Zellteilung hemmende (antiproliferative) Wirkstoffe, Zellen rekrutierende Wirkstoffe, zelladhäsive Wirkstoffe, Proteine, Peptide, Cytokine, Polysaccharide, Mucopolysaccharide, Glykoproteine, Nukleinsäuren, Salze davon und Kombinationen davon.

Das Produkt, insbesondere die schaumartige Komponente und/oder die membranartige Komponente, kann - abhängig von der jeweils zu behandelnden Indikation - in unterschiedlichen Formen und Größen vorliegen. Beispielsweise kann das medizinische Produkt, insbesondere die schaumartige Komponente und/oder die membranartige Komponente, scheibenförmig, insbesondere als kreisförmige Scheibe, zylindrisch, insbesondere als Flachzylinder, keil- oder streifenförmig ausgebildet sein.

Insbesondere kann das Produkt, insbesondere die schaumartige Komponente und/oder die membranartige Komponente, einen gekrümmten oder kurvenförmigen Verlauf aufweisen.

Bevorzugt ist das medizinische Produkt, insbesondere die schaumartige Komponente und/oder die membranartige Komponente, halbkreisförmig, C-förmig, sichelförmig oder halbmondförmig ausgebildet.

Besonders bevorzugt besitzt das medizinische Produkt, insbesondere die schaumartige Komponente und/oder die membranartige Komponente, die Form einer gekrümmten oder kurvenförmigen, vorzugsweise halbkreisförmigen, C-förmigen, sichelförmigen oder halbmondförmigen, Scheibe oder eines gekrümmten oder kurvenförmigen, vorzugsweise halbkreisförmigen, C-förmigen, sichelförmigen oder halbmondförmigen, Keils. In diesen Ausführungsformen eignet sich das medizinische Produkt insbesondere als Ersatz- oder Teilersatzimplantat für Menisken wie beispielsweise den Innen- und/oder Außenmeniskus eines Kniegelenks.

Die vorliegende Erfindung umfasst weiterhin ein Verfahren zur Herstellung eines medizinischen Produktes gemäß der vorliegenden Erfindung. Bei dem Verfahren wird ein Treibmittel enthaltendes Reaktionsgemisch für eine schaumartige Komponente in Gegenwart einer membranartigen Komponente und einer faserförmigen Komponente aufschäumen gelassen.

Bevorzugt erfolgt die Herstellung mittels eines Reaktionsschaumguss-Verfahrens bzw. nach Art eines Reaktionsschaumguss-Verfahrens.

Das Verfahren wird vorzugsweise in einem eine Kavität aufweisenden Formwerkzeug, beispielsweise aus Silikon (Silikonform), durchgeführt. Das Formwerkzeug besitzt vorzugsweise zwei Formwerkzeugteile, in der Regel ein Formwerkzeug-Oberteil und ein Formwerkzeug-Unterteil, welche die Kavität bilden. Die Kavität des Formwerkzeuges bestimmt das Volumen und die Form der schaumartigen Komponente.

Das medizinische Produkt wird in einer weitergehenden Ausführungsform hergestellt, indem
a) das Formwerkzeug geöffnet und die membranartige Komponente in das geöffnete Formwerkzeug eingelegt wird, vorzugsweise derart, dass die membranartige Komponente nach Schließen des Formwerkzeuges in die Kavität des Formwerkzeuges hineinragt,
b) das Formwerkzeug geschlossen wird,
c) das Treibmittel enthaltende Reaktionsgemisch und die faserförmige Komponente in die Kavität des Formwerkzeuges eingebracht werden,
d) das Reaktionsgemisch aufschäumen gelassen wird,
e) das Produkt entformt wird.

In einer bevorzugten Ausführungsform wird die membranartige Komponente vor dem Aufschäumen lassen des Reaktionsgemisches, insbesondere vor dem Einlegen in das geöffnete Formwerkzeug, mit Durchbrechungen, insbesondere Öffnungen, Löcher, Perforationen oder dergleichen, versehen. Die Durchbrechungen können beispielsweise durch Einschneiden oder Ausstanzen der Membran erzeugt werden. Die Durchbrechungen stellen sicher, dass der Aufschäumprozess durch eine in die Kavität des Formwerkzeuges hineinragende membranartige Komponente nicht beeinträchtigt wird und somit die gesamte Kavität des Formwerkzeuges zum Aufschäumen genutzt werden kann.

Das Formwerkzeug wird in einer weiteren Ausführungsform vor dem Einlegen der membranartigen Komponente temperiert, bevorzugt auf eine Temperatur zwischen 35 und 60 °C, insbesondere 40 und 55 °C, bevorzugt auf eine Temperatur von ca. 50 °C. Die Temperierung kann beispielsweise während eines Zeitraumes von 0,5 bis 2 Stunden, bevorzugt während eines Zeitraumes von ca. 1 Stunde, vorgenommen werden.

In einer besonders bevorzugten Ausführungsform wird ein Teil der membranartigen Komponente zwischen zwei Formwerkzeugteilen, bevorzugt zwischen einem Formwerkzeug-Oberteil und einem Formwerkzeug-Unterteil, vorzugsweise außerhalb der Kavität fixiert. Zur Fixierung dieses Teils der membranartigen Komponente können die Formwerkzeugteile jeweils eine Anlagefläche aufweisen, welche sich nach dem Schließen des Formwerkzeuges gegenüberliegen. In der Regel besitzen die Anlageflächen der Formwerkzeugteile eine Größe, welche in etwa der Größe des zu fixierenden Teils der membranartigen Komponente entspricht. Die Fixierung der membranartigen Komponente in dem Formwerkzeug kann beispielsweise mittels geeigneter Fixierhilfen wie beispielsweise Kanülennadeln oder dergleichen realisiert oder zusätzlich unterstützt werden.

Das Einbringen des Treibmittel enthaltenden Reaktionsgemisches und der faserförmigen Komponente in das Formwerkzeug erfolgt in der Regel über hierfür vorgesehene Öffnungen des Formwerkzeuges, beispielsweise mittels Injektion.

Grundsätzlich können das Treibmittel enthaltende Reaktionsgemisch und die faserförmige Komponente als getrennte Komponenten in das Formwerkzeug eingebracht werden. Bevorzugt ist es jedoch, wenn die faserförmige Komponente bereits in dem Treibmittel enthaltenden Reaktionsgemisch enthalten ist.

Als Treibmittel für das Reaktionsgemisch können grundsätzlich physikalische und/oder chemische Treibmittel verwendet werden. Geeignete Treibmittel können zum Beispiel aus der Gruppe bestehend aus Wasser, Kohlendioxid, Luft, Stickstoff, Stickoxide, Edelgase, Fluorkohlenwasserstoffe, Carbamate, Methylal oder Mischungen davon ausgewählt werden.

Bevorzugt enthält das Reaktionsgemisch neben dem Treibmittel wenigstens eine Polyolkomponente und wenigstens eine Polyisocyanatkomponente, vorzugsweise wenigstens eine Diolkomponente und wenigstens eine Diisocyanatkomponente.

Als wenigstens eine Polyolkomponente können beispielsweise Polyole aus der Gruppe bestehend aus Ethylenglykol, Diethylenglykol, Propylenglykol, Trimethylolpropan, Glycerin, Neopentylglykol, 1,3-Propandiol, 1,4-Butandiol, 1,6-Hexandiol, Polycaprolactondiol, Polycaprolactontriol, Polyethylenglykol und Mischungen davon verwendet werden.

Erfindungsgemäß kann es insbesondere vorgesehen sein, eine Polyolkomponentenmischung (Polyolmischung) zu verwenden.

Als wenigstens eine Polyisocyanatkomponente können beispielsweise Polyisocyanate verwendet werden, welche aus der Gruppe bestehend aus Diphenylmethandiisocyanat, Toluol-2,4-Diisocyanat, Naphthylendiisocyanat, Hexamethylendiisocyanat, Isophorondiisocyanat, 4,4'-Diisocyanatodicyclohexylmethan, Butandiisocyanat, Lysindiisocyanat und Mischungen davon ausgewählt sind.

In einer weiteren Ausführungsform wird die Polyolkomponente vor dem Einbringen in das Formwerkzeug verflüssigt, beispielsweise mittels Temperieren auf eine Temperatur zwischen 30 und 50 °C, insbesondere 35 und 45 °C, bevorzugt auf eine Temperatur von ca. 40 °C.

Das Treibmittel enthaltende Reaktionsgemisch kann ferner einen Katalysator, insbesondere ein Amin, vorzugsweise ein tertiäres Amin, enthalten. Bei dem Katalysator handelt es sich bevorzugt um 1,4-Diazabicyclo[2.2.2]octan, Diazabicycloundecen oder eine Mischung davon. Außerdem können als Katalysator auch zinnorganische Verbindungen wie beispielsweise Dibutylzinndilaurat verwendet werden.

Gegebenenfalls kann das Treibmittel enthaltende Reaktionsgemisch weitere Additive wie beispielsweise Schaumstabilisatoren, insbesondere ionische Emulgatoren, enthalten. Durch Schaumstabilisatoren kann in vorteilhafter Weise die Porengrösse reguliert und/oder die Porosität, insbesondere Offenporigkeit, beeinflusst werden. Als geeignete Schaumstabilisatoren können zum Beispiel Lecithin und/oder bestimmte Silikon-Polyether-Copolymere verwendet werden.

Weiterhin kann es bevorzugt sein, wenn die membranartige Komponente vor dem Einlegen in das Formwerkzeug in das Reaktionsgemisch ohne das Treibmittel, vorzugsweise in ein Gemisch, enthaltend eine Polyolkomponente und eine Polyisocyanatkomponente, eingelegt wird. Dadurch lässt sich mit besonderem Vorteil das Haftvermögen der membranartigen Komponente an dem Formwerkzeug bzw. an Formwerkzeugteilen erhöhen.

Das Formwerkzeug wird in einer weiteren Ausführungsform nach dem Aufschäumen des Reaktionsgemisches und vorzugsweise vor dem Entformen des Produktes getempert, bevorzugt auf eine Temperatur zwischen 40 und 60 °C, insbesondere 45 und 55 °C.

Weiterhin kann es von Vorteil sein, dass das Formwerkzeug vor dem Entformen des Produktes abgekühlt wird.

In einer weiteren Ausführungsform werden nach dem Entformen des Produktes Kanäle bzw. kanalartige Strukturen in der schaumartigen Komponente ausgebildet. Dies kann beispielsweise mittels einer Kunststoffschiene mit abstehenden Kunststoffspitzen oder einer Metallschiene mit abstehenden Metallspitzen erfolgen.

Alternativ kann zur Ausbildung von Kanälen bzw. kanalartigen Strukturen in der schaumartigen Komponente ein Formwerkzeug mit integrierten borstenartigen Strukturen, welche zweckmäßigerweise in die Kavität des Formwerkzeuges hineinragen, verwendet werden.

Bei dem medizinischen Produkt gemäß der vorliegenden Erfindung handelt es sich vorzugsweise um ein Implantat, insbesondere chirurgisches Implantat.

Weiterhin bevorzugt ist es, wenn das medizinische Produkt als Implantat zur Regeneration und/oder Rekonstruktion von Gewebe, insbesondere Bindegewebe, bevorzugt Stützgewebe, weiter bevorzugt Knorpelgewebe, besonders bevorzugt Meniskusgewebe, ausgebildet ist.

Bevorzugt handelt es sich bei dem medizinischen Produkt um ein Knorpelersatz- oder Knorpelteilersatzimplantat. Bei dem zu ersetzenden Knorpel kann es sich um Meniskus-Knorpel und/oder HNO-Knorpel wie beispielsweise Knorpel der Ohrmuschel, des äußeren Gehörganges, der Ohrtrompete, des Kehldeckels und/oder der kleinen Bronchien handeln.

Das medizinische Produkt ist in einer weiteren Ausführungsform als Ersatz- oder Teilersatzimplantat für Bandscheibendefekte ausgebildet.

Besonders bevorzugt ist das medizinische Produkt als Meniskusersatz- oder Meniskusteilersatzimplantat, insbesondere für Gelenke, vorzugsweise für das Kniegelenk, ausgebildet. So ist es erfindungsgemäß insbesondere bevorzugt, wenn es sich bei dem medizinischen Produkt um ein Ersatz- oder Teilersatzimplantat für den Innen- und/oder Außenmeniskus eines menschlichen und/oder tierischen Kniegelenks handelt.

Eine weitere Verwendung des medizinischen Produktes betrifft seine Verwendung als Träger, insbesondere Trägerimplantat, für Zellen, insbesondere autologe Zellen, bevorzugt autologe Körperzellen. Bezüglich der in Frage kommenden Zellen wird auf die vorherigen Ausführungen Bezug genommen. In dieser Form eignet sich das medizinische Produkt in besonders vorteilhafter Weise als regeneratives Implantat bzw. Konstrukt, d.h. als Implantat bzw. Konstrukt, welches die Rekonstruktion und/oder Regeneration von beschädigtem Gewebe fördert.

Des Weiteren kann das medizinische Produkt als Wirkstofffreisetzungssystem (drug delivery device) verwendet werden. Bezüglich der in Frage kommenden Wirkstoffe wird ebenfalls auf die vorherigen Ausführungen verwiesen.

Weitere Merkmale und Vorteile der Erfindung ergeben sich aus der nachfolgenden Beschreibung bevorzugter Ausführungsformen in Kombination mit den Figuren, den Figurenbeschreibungen, dem Beispiel und den Unteransprüchen. In diesen Ausführungsformen können einzelne Merkmale der Erfindung alleine oder in Kombination mit anderen Merkmalen verwirklicht sein. Die beschriebenen bevorzugten Ausführungsformen sind lediglich als beschreibende, keineswegs als in irgendeiner Weise limitierende Offenbarung zu verstehen. Sämtliche Figuren werden hiermit durch ausdrückliche Bezugnahme zum Inhalt dieser Beschreibung gemacht.

In den Figuren zeigen schematisch:
- Fig. 1: eine perspektivische Draufsicht auf eine Ausführungsform des medizinischen Produktes und
- Fig. 2: eine Seitenansicht der in Figur 1 dargestellten Ausführungsform.

### 1. Herstellung eines erfindungsgemäßen Produktes zum Ersatz oder Teilersatz eines Meniskus

### 1.1 Vorbereitung der Rinderperikard-Membran

Die Membran wurde mit einer Kanüle (21 G x 2) perforiert. Anschließend wurde die Membran im Vakuum (2-10 mbar) für wenigstens sechs Stunden getrocknet. Danach wurde die Membran in eine Mischung aus Polycaprolactondiol (Mₙ=2000) und Isophorondiisocyanat im molaren Verhältnis 9:10 bei 50 °C eingelegt. Nach 90 Minuten wurden die Membran und die Mischung in ein Polyethylen-Wägeschiffchen überführt, in welchem die Membran mittels eines PTFE-ummantelten Rührfischchens fixiert wurde. Dort verblieb die Membran für insgesamt zwei Stunden, ehe die Membran in eine zweiteilige Silikonform (Formwerkzeug) eingelegt wurde.

### 1.2 Einlegen der Perikardmembran in ein Formwerkzeug

Eine zweiteilige Silikonform mit einer halbkreisförmigen Kavität wurde vor dem Einlegen der Perikardmembran für mindestens eine Stunde auf 50 °C temperiert. Anschließend wurde die Membran in die Silikonform eingelegt und mit Hilfe von drei Kanülen (21 G x 2) fixiert. Danach wurde die Silikonform von außen mit einem Klebeband verklebt.

### 1.3 Herstellung einer Polyolmischung

Die Polyole Polycaprolactondiol, Polycaprolactontriol, Polyethylenglykol und Butandiol wurden zur Verflüssigung auf eine Temperatur von ca. 40 °C temperiert, um sie zu verflüssigen. Danach wurden die Polyole in einem geeigneten, ebenfalls auf 40 °C temperierten Gefäß eingewogen und vermischt. Anschließend wurden die Additive DABCO, DC3042 (Silikon-Polyether-Copolymer, bei welchem sich die Polyethergruppen in Seitengruppen des Silikons befinden) und Lecithin hinzugegeben. Danach wurde eine homogene Mischung hergestellt. Schließlich wurden zu der Mischung kollagenhaltige Fasern aus der Muskelschicht des Herzens (Myokard) hinzugegeben. Die erhaltene Mischung wurde erneut homogenisiert.

### 1.4 Zugabe der weiteren Komponenten

Zu der gemäß 1.3. hergestellten Mischung wurde Isophorondiisocyanat und nach kurzem Homogenisieren Methylal als Treibmittel hinzugegeben. Nach der Homogenisierung der Mischung erfolgte die Zugabe von Diazabicycloundecen.

Als die Mischung zu schäumen begann, wurde sie mittels einer 50 ml Spritze in die Silikonform injiziert. Hierzu wies die Silikonform geeignete Injektionsöffnungen auf.

Die Silikonform wurde für wenigstens fünf Stunden bei ca. 50 °C im Wärmeschrank gelagert. Danach ließ man die geschlossene Form für wenigstens zwei Stunden abkühlen.

Anschließend wurde die Siliconform geöffnet und das fertige Produkt vorsichtig entformt. Überschüssiges Polyurethan (Angüsse, Material in den Entlüftungskanälen, poröser Film im Innern des Halbkreissegmentes des Produktes) wurde entfernt. Das Produkt wurde in voll entsalztem Wasser für ca. zwei Minuten gewalkt und danach im Vakuum bis zur Gewichtskonstanz getrocknet.

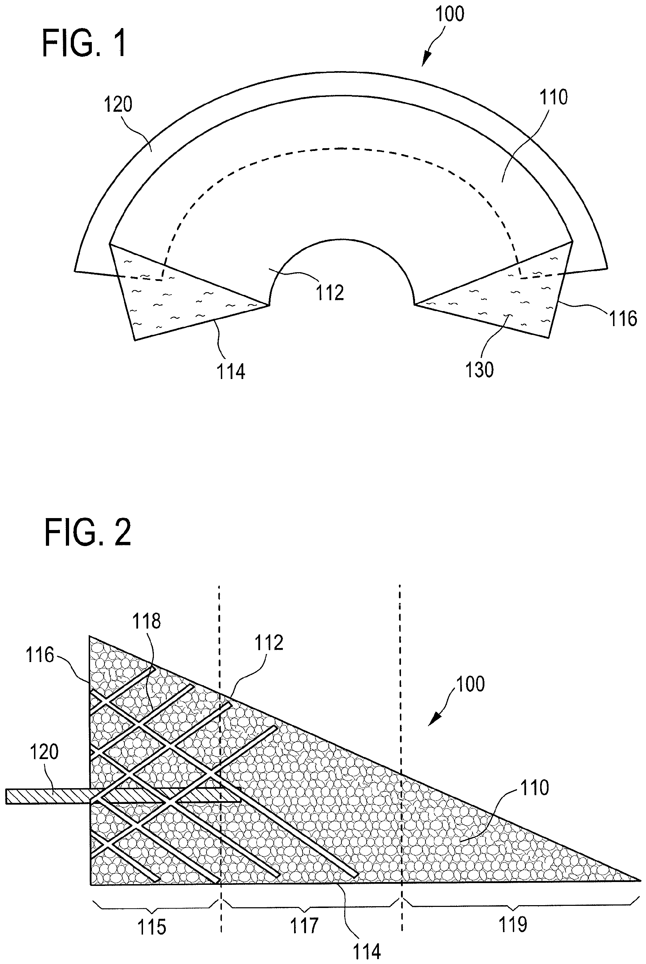

Das erhaltene Produkt besaß eine Form, wie sie schematisch in der Fig. 1 dargestellt ist.

### 2. Figurenbeschreibungen

Fig. 1 zeigt schematisch eine besonders bevorzugte Ausführungsform für ein medizinisches Produkt 100 gemäß der vorliegenden Erfindung. Das medizinische Produkt 100 weist eine schaumartige Komponente 110, eine membranartige Komponente 120 und eine faserförmige Komponente 130 auf.

Die schaumartige Komponente 110 besitzt in der Regel eine offenporöse Struktur und dient vorzugsweise als Trägermatrix für einwachsende bzw. einsprossende Zellen wie beispielsweise Chondrozyten, Vorläuferzellen davon und/oder Stammzellen davon.

Bei der schaumartigen Komponente 110 handelt es sich bevorzugt um ein geschäumtes Polymer bzw. um einen Polymerschaum, insbesondere um geschäumtes Polyurethan bzw. einen Polyurethanschaum.

Die schaumartige Komponente 110 besitzt - wie in Figur 1 schematisch dargestellt - bevorzugt die Form eines halbkreisförmigen oder C-förmigen Keils. Darüber hinaus sind jedoch, abhängig von der jeweiligen medizinischen Indikation, auch andere Formen denkbar.

Das in der Figur 1 (und der Figur 2) schematisch dargestellte Produkt 100 eignet sich insbesondere als Meniskusersatz- oder Meniskusteilersatzimplantat, bevorzugt als Ersatz- oder Teilersatzimplantat für den Innen- und/oder Außenmeniskus des Kniegelenks, vorzugsweise des menschlichen Kniegelenks.

Bevorzugt ist die Oberfläche 112 nach Implantation des Produktes 100 in ein Kniegelenk den Oberschenkelkondylen (femorale Kondylen) zugewandt (oberschenkelseitige Oberfläche). Die Oberfläche 114 ist nach Implantation des Produktes 100 in ein Kniegelenk vorzugsweise den Schienbeinkondylen (tibiale Kondylen) zugewandt (schienbeinseitige Oberfläche). Die Oberfläche 116 ist nach Implantation des Produktes 100 vorzugsweise der Gelenkkapsel eines Kniegelenks zugewandt (gelenkkapselseitige Oberfläche).

Die membranartige Komponente 120 ragt entlang der Oberfläche 116 aus der schaumartigen Komponente 110 heraus.

Die membranartige Komponente 120 besitzt vorzugsweise im Wesentlichen den gleichen gekrümmten Verlauf wie die schaumartige Komponente 110.

Die membranartige Komponente 120 ist eine tierische Membran wie beispielsweise Rinderperikard.

Bei der faserförmigen Komponente 130 handelt es sich bevorzugt um proteinhaltige, insbesondere kollagenhaltige, Fasern wie beispielsweise Myokardfasern. Die faserförmige Komponente 130 ist vorzugsweise ausschließlich in der schaumartigen Komponente 110 enthalten.

Wie bereits in der bisherigen Beschreibung ausführlich ausgeführt, können sowohl die membranartige Komponente 120 als auch die faserförmige Komponente 130 mit besonderem Vorteil als Leit- bzw. Führungsschienen für Zellen, Körpergewebe und/oder natürliche Versorgungsgefäße in die schaumartige Komponente 110 wirken. Dadurch ist eine rasche, quantitative und insbesondere homogene zelluläre Besiedelung der schaumartigen Komponente 110, eine bindegewebsbasierte Verankerung des Produktes 100 in einer Körperdefektzone und/oder eine ausreichende Nährstoffversorgung möglich. Dies begünstigt, insbesondere beschleunigt, die Neuenstehung von Zielgewebe, vorzugsweise Knorpelgewebe, in einer zu versorgenden Körperdefektzone, vorzugsweise Knorpeldefektzone.

Um eine zelluläre Einsprossung, Gewebeeinsprossung und/oder Gefäßeinsprossung in die schaumartige Komponente 110 zusätzlich zu verbessern, kann die schaumartige Komponente 110 Kanäle bzw. kanalartige Strukturen 118 aufweisen (Figur 2, faserförmige Komponente ist in der Figur 2 nicht dargestellt). Beispielsweise können die Kanäle 118 lediglich in einem äußeren Bereich 115 und einem mittleren Bereich 117 der schaumartigen Komponente 110 ausgebildet sein, während ein zentraler Bereich 119 der schaumartigen Komponente 110 vorzugsweise frei von derartigen Kanälen 118 ist. Dadurch besitzt der zentrale Bereich 119 eine im Verhältnis zum peripheren Bereich 115 und mittleren Bereich 117 kompaktere Struktur, was insbesondere bei einem als Meniskusersatz- oder -teilersatzimplantat ausgebildeten Produkt 100 von Vorteil ist, bei welchem der zentrale Bereich 119 besonders hohen Belastungskräften, insbesondere Scherkräften, ausgesetzt ist.

## Patentansprüche

1. Medizinisches Produkt (100), insbesondere zur Anwendung bei der Behandlung von Knorpelschäden und/oder -defekten, umfassend eine schaumartige Komponente (110), eine membranartige Komponente (120) und eine faserförmige Komponente (130), wobei es sich bei der membranartigen Komponente (120) um eine tierische Membran handelt und die membranartige Komponente (120) teilweise in der schaumartigen Komponente (110) enthalten ist.

2. Medizinisches Produkt (100) nach Anspruch 1, **dadurch gekennzeichnet, dass** es sich bei der schaumartige Komponente (110) um ein geschäumtes Polymer, insbesondere um ein geschäumtes synthetisches Polymer, handelt.

3. Medizinisches Produkt (100) nach Anspruch 1 oder 2, **dadurch gekennzeichnet, dass** es sich bei der schaumartigen Komponente (110) um geschäumtes Polyurethan bzw. einen Polyurethanschaum handelt.

4. Medizinisches Produkt (100) nach einem der vorhergehenden Ansprüche, **dadurch gekennzeichnet, dass** die schaumartige Komponente (110) Kanäle bzw. kanalartige Strukturen (118), vorzugsweise zum Einwachsen von Zellen, Gewebe und/oder natürlichen Versorgungsgefäßen wie beispielsweise Blutgefäßen, aufweist, wobei die Kanäle bzw. kanalartigen Strukturen (118) vorzugsweise nur in einem peripheren Bereich (115) der schaumartigen Komponente (110) und gegebenenfalls einem daran angrenzenden mittleren Bereich (117) der schaumartigen Komponente (110) ausgebildet sind.

5. Medizinisches Produkt (100) nach einem der vorhergehenden Ansprüche, **dadurch gekennzeichnet, dass** die membranartige Komponente (120) nur teilweise in der schaumartigen Komponente (110) enthalten ist.

6. Medizinisches Produkt (100) nach einem der vorhergehenden Ansprüche, **dadurch gekennzeichnet, dass** die schaumartige Komponente (110) eine oberschenkelseitige Oberfläche (112), eine schienbeinseitige Oberfläche (114) und eine gelenkkapselseitige Oberfläche (116) aufweist, wobei die membranartige Komponente (120) vorzugsweise entlang der gelenkkapselseitigen Oberfläche (116) herausragt.

7. Medizinisches Produkt (100) nach einem der vorhergehenden Ansprüche, **dadurch gekennzeichnet, dass** die membranartige Komponente (120) Kollagen, insbesondere faserförmiges bzw. fibrilläres Kollagen, vorzugsweise ausgewählt aus der Gruppe bestehend aus Kollagen vom Typ I, Kollagen vom Typ II, Kollagen vom Typ III, Kollagen vom Typ V, Kollagen vom Typ XI, Derivate davon, Salze davon und Mischungen davon aufweist.

8. Medizinisches Produkt (100) nach einem der vorhergehenden Ansprüche, **dadurch gekennzeichnet, dass** es sich bei der membranartigen Komponente (120) um Perikard, vorzugsweise um bovines Perikard, handelt.

9. Medizinisches Produkt (100) nach einem der vorhergehenden Ansprüche, **dadurch gekennzeichnet, dass** die faserförmige Komponente (130) ausschließlich in der schaumartigen Komponente (110) enthalten ist.

10. Medizinisches Produkt (100) nach einem der vorhergehenden Ansprüche, **dadurch gekennzeichnet, dass** die faserförmige Komponente (130) aus dem gleichen Material gebildet ist wie die membranartige Komponente (120).

11. Medizinisches Produkt (100) nach einem der vorhergehenden Ansprüche, **dadurch gekennzeichnet, dass** es sich bei der faserförmigen Komponente (130) um proteinhaltige Fasern, insbesondere kollagenhaltige Fasern, bevorzugt Myokardfasern, handelt.

12. Medizinisches Produkt (100) nach einem der vorhergehenden Ansprüche, **dadurch gekennzeichnet, dass** das Produkt (100), insbesondere die schaumartige Komponente (110) und/oder die membranartige Komponente (120), Zellen, insbesondere autologe Körperzellen, vorzugsweise ausgewählt aus der Gruppe bestehend aus Chondrozyten, Osteoblasten, Zellen aus dem Periostgewebe, Zellen aus dem Perichondriumgewebe, Fibroblasten, Vorläuferzellen davon, Stammzellen davon und Mischungen davon, aufweist.

13. Medizinisches Produkt (100) nach einem der vorhergehenden Ansprüche, **dadurch gekennzeichnet, dass** das Produkt (100), insbesondere die schaumartige Komponente (110) und/oder die membranartige Komponente (120), halbkreis-, halbmond-, sichel- oder C-förmig ausgebildet ist, bevorzugt als Keil oder Scheibe mit halbkreis-, halbmond-, sichel- oder C-förmigem Verlauf vorliegt.

14. Verfahren zur Herstellung eines medizinischen Produktes nach einem der vorhergehenden Ansprüche, **dadurch gekennzeichnet, dass** ein Treibmittel enthaltendes Reaktionsgemisch für eine schaumartige Komponente in Gegenwart einer membranartigen Komponente und einer faserförmigen Komponente, vorzugsweise in einem eine Kavität aufweisenden Formwerkzeug, aufschäumen gelassen wird.

15. Medizinisches Produkt (100) nach einem der Ansprüche 1 bis 13 als Implantat, insbesondere als Knorpelersatz- oder Knorpelteilersatzimplantat, bevorzugt als Meniskusersatz- oder Meniskusteilersatzimplantat, besonders bevorzugt zum Ersatz oder Teilersatz des Außen- und/oder Innenmeniskus eines menschlichen oder tierischen Kniegelenks.

## Claims

1. Medical product (100), in particular for use in the treatment of cartilage lesions and/or cartilage defects, comprising a foam-type component (110), a membrane-type component (120) and a fibrous component (130), wherein the membrane-type component (120) is an animal membrane and the membrane-type component (120) is partially included in the foam-type component (110).

2. Medical product (100) according to claim 1, **characterized in that** the foam-type component (110) is a foamed polymer, in particular a foamed synthetic polymer.

3. Medical product (100) according to claim 1 or 2, **characterized in that** the foam-type component (110) is foamed polyurethane or a polyurethane foam.

4. Medical product (100) according to any one of the preceding claims, **characterized in that** the foam-type component (110) has channels or channel-type structures (118), preferably for the ingrowth of cells, tissue and/or natural supply vessels, like blood vessels, for example, wherein the channels or channel-type structures (118) preferably are provided only in a peripheral region (115) of the foam-type component (110) and, optionally, in a central region (117) of the foam-type component (110) adjoining thereto.

5. Medical product (100) according to any one of the preceding claims, **characterized in that** the membrane-type component (120) is only partially included in the foam-type component (110).

6. Medical product (100) according to any one of the preceding claims, **characterized in that** the foam-type component (110) has a femur-sided surface (112), a tibia-sided surface (114) and a joint capsule-sided surface (116), wherein the membrane-type component (120) preferably projects along the joint capsule-sided surface (116).

7. Medical product (100) according to any one of the preceding claims, **characterized in that** the membrane-type component (120) includes collagen, in particular fibrous or fibrillar collagen, preferably selected from the group consisting of type I collagen, type II collagen, type III collagen, type V collagen, type XI collagen, derivatives thereof, salts thereof and mixtures thereof.

8. Medical product (100) according to any one of the preceding claims, **characterized in that** the membrane-type component (120) is pericardium, preferably bovine pericardium.

9. Medical product (100) according to any one of the preceding claims, **characterized in that** the fibrous component (130) is included exclusively in the foam-type component (110).

10. Medical product (100) according to any one of the preceding claims, **characterized in that** the fibrous component (130) is composed of the same material as the membrane-type component (120).

11. Medical product (100) according to any one of the preceding claims, **characterized in that** the fibrous component (130) is protein-containing fibres, in particular collagen-containing fibres, preferably myocardium fibres.

12. Medical product (100) according to any one of the preceding claims, **characterized in that** the product (100), in particular the foam-type component (110) and/or the membrane-type component (120), includes cells, in particular autologous somatic cells, preferably selected from the group consisting of chondrocytes, osteoblasts, cells from periosteum tissue, cells from perichondrium tissue, fibroblasts, precursor cells thereof, stem cells thereof and mixtures thereof.

13. Medical product (100) according to any one of the preceding claims, **characterized in that** the product (100), in particular the foam-type component (110) and/or the membrane-type component (120), has a semicircular, crescent, falcate or C-type shape, preferably is a wedge or disk presenting a semicircular, crescent, falcate or C-type shaped pattern.

14. Method for the production of a medical product according to any one of the preceding claims, **characterized in that** a reactive composition containing a blowing agent for a foam-type component is allowed to foam in the presence of a membrane-type component and a fibrous component, preferably in a moulding tool having a cavity.

15. Medical product (100) according to any of claims 1 to 13 in the form of an implant, in particular an implant for replacement of cartilage or partial replacement of cartilage, preferably an implant for replacement of meniscus or partial replacement of meniscus, particularly preferred for replacement or partial replacement of the outer meniscus and/or inner meniscus of a knee joint in a human or an animal.

## Revendications

1. Produit médical (100), en particulier pour l'utilisation dans le traitement de lésions de cartilage et/ou de défauts de cartilage, comportant un composant de type mousse (110), un composant de type membrane (120) et un composant fibreux (130), dans lequel le composant de type membrane (120) est une membrane animale et le composant de type membrane (120) est partiellement contenu dans le composant de type mousse (110).

2. Produit médical (100) selon la revendication 1, **caractérisé en ce que** le composant de type mousse (110) est un polymère expansé, en particulier un polymère synthétique expansé.

3. Produit médical (100) selon la revendication 1 ou 2, **caractérisé en ce que** le composant de type mousse (110) est du polyuréthane expansé ou une mousse de polyuréthane.

4. Produit médical (100) selon l'une quelconque des revendications précédentes, **caractérisé en ce que** le composant de type mousse (110) présente des canaux ou des structures de type canal (118), de préférence pour la croissance interne de cellules, de tissu et/ou des vaisseaux d'alimentation naturels, comme par exemple des vaisseaux sanguins, dans lequel les canaux ou les structures de type canal (118) sont formés de préférence seulement dans une zone périphérique (115) du composant de type mousse (110) et le cas échéant dans une zone centrale (117) adjacente du composant de type mousse (110).

5. Produit médical (100) selon l'une quelconque des revendications précédentes, **caractérisé en ce que** le composant de type membrane (120) est seulement partiellement contenu dans le composant de type mousse (110).

6. Produit médical (100) selon l'une quelconque des revendications précédentes, **caractérisé en ce que** le composant de type mousse (110) présente une surface du côté fémur (112), une surface du côté tibia (114) et une surface du côté capsule articulaire (116), dans lequel le composant de type membrane (120) dépasse de préférence le long de la surface du côté capsule articulaire (116).

7. Produit médical (100) selon l'une quelconque des revendications précédentes, **caractérisé en ce que** le composant de type membrane (120) comporte du collagène, en particulier du collagène fibreux ou fibrillaire, de préférence sélectionné dans le groupe composé du collagène de type I, collagène de type II, collagène de type III, collagène de type V, collagène de type XI, des dérivés de ceux-ci, des sels de ceux-ci et des mixtures de ceux-ci.

8. Produit médical (100) selon l'une quelconque des revendications précédentes, **caractérisé en ce que** le composant de type membrane (120) est du péricarde, de préférence du péricarde bovin.

9. Produit médical (100) selon l'une quelconque des revendications précédentes, **caractérisé en ce que** le composant fibreux (130) est contenu exclusivement dans le composant de type mousse (110).

10. Produit médical (100) selon l'une quelconque des revendications précédentes, **caractérisé en ce que** le composant fibreux (130) se compose du même matériau que le composant de type membrane (120).

11. Produit médical (100) selon l'une quelconque des revendications précédentes, **caractérisé en ce que** le composant fibreux (130) est des fibres contenant de la protéine, en particulier des fibres contenant du collagène, de préférence des fibres myocardiques.

12. Produit médical (100) selon l'une quelconque des revendications précédentes, **caractérisé en ce que** le produit (100), en particulier le composant de type mousse (110) et/ou le composant de type membrane (120), comporte des cellules, en particulier des cellules somatiques autologues, de préférence sélectionnées dans le groupe composé de chondrocytes, ostéoblastes, cellules du tissu périostique, cellules du tissu périchondrique, fibroblastes, cellules précurseurs de ceux-ci, cellules souches de ceux-ci et des mixtures de ceux-ci.

13. Produit médical (100) selon l'une quelconque des revendications précédentes, **caractérisé en ce que** le produit (100), en particulier le composant de type mousse (110) et/ou le composant de type membrane (120), se présente sous forme semi-circulaire, de demi-lune, de faucille ou de C, de préférence sous forme d'un coin ou d'un disque en formation semi-circulaire, en demi-lune, en faucille ou en C.

14. Procédé de fabrication d'un produit médical selon l'une quelconque des revendications précédentes, **caractérisé en ce qu'**on laisse s'expanser une mélange de réaction contenant un agent gonflant pour un composant de type mousse en présence d'un composant de type membrane et un composant fibreux, de préférence dans un outil de moulage présentant une cavité.

15. Produit médical (100) selon l'une quelconque des revendications 1 à 13, sous forme d'implant, en particulier sous forme d'implant de remplacement de cartilage ou d'implant de remplacement partiel de cartilage, de préférence sous forme d'implant de remplacement de ménisque ou d'implant de remplacement partiel de ménisque, particulièrement préféré pour le remplacement ou le remplacement partiel du ménisque extérieur et/ou du ménisque intérieur d'une articulation du genou humain ou animal.
